# EUROPÄISCHE PATENTSCHRIFT

(11) **EP 3 307 389 B1**
(45) Veröffentlichungstag und Bekanntmachung des Hinweises auf die Patenterteilung: **20.12.2023**
(21) Anmeldenummer: 16750603.9
(22) Anmeldetag: 10.06.2016
(51) Int. Cl.: A61K 31/202, A61P 15/08, G01N 33/00

(54) **OMEGA-3- UND/ODER OMEGA-6-FETTSÄURE(N) BEI BEEINTRÄCHTIGTEM SPHINGOLIPID-METABOLISMUS**
OMEGA-3 AND/OR OMEGA-6 FATTY ACID(S) IN IMPAIRED SPHINGOLIPID METABOLISM
UTILISATION D'ACIDE(S) GRAS OMÉGA-3 ET/OU OMÉGA-6 DANS LE TRAITEMENT DES TROUBLES DU MÉTABOLISME DES SPHINGOLIPIDES

(30) Priorität: 12.06.2015 DE 102015109352
(43) Veröffentlichungstag der Anmeldung: 18.04.2018
(73) Patentinhaber: K.D. Pharma Bexbach GMBH, 66450 Bexbach (DE)
(72) Erfinder: HARTMANN, Tobias, 66909 Matzenbach (DE); GRIMM, Marcus Otto Walter, 66424 Homburg (DE); FLOSS, Thomas, 80636 München (DE); WITTMANN, Anke, 80637 München (DE)
(74) Vertreter: Patentanwälte Bernhardt / Wolff Partnerschaft mbB
(86) Internationale Anmeldenummer: PCT/DE2016/100265
(87) Internationale Veröffentlichungsnummer: WO 2016/198048

(56) Entgegenhaltungen:
- PACHECO FABIO J ET AL: "Docosahexanoic acid antagonizes TNF-[alpha]-induced necroptosis by attenuating oxidative stress, ceramide production, lysosomal dysfunction, and autophagic feat", INFLAMMATION RESEARCH, BIRKHAEUSER VERLAG, BASEL, CH, Bd. 63, Nr. 10, 6. August 2014 (2014-08-06), Seiten 859-871, XP035388462, ISSN: 1023-3830, DOI: 10.1007/S00011-014-0760-2 [gefunden am 2014-08-06]
- Mike Barrett: "Omega-3 Fats Provide Powerful Fertility Benefits", Natural Society , 17. Januar 2012 (2012-01-17), XP055310676, Gefunden im Internet: URL:about:reader?url=http://naturalsociety .com/omega-3-fats-provide-powerful-fertili ty-benefits/ [gefunden am 2016-10-13]
- SAFARINEJAD M R ET AL: "Relationship of omega-3 and omega-6 fatty acids with semen characteristics, and anti-oxidant status of seminal plasma: A comparison between fertile and infertile men", CLINICAL NUTRITION, CHURCHILL LIVINGSTONE, LONDON, GB, vol. 29, no. 1, 1 February 2010 (2010-02-01), pages 100-105, XP026880343, ISSN: 0261-5614, DOI: 10.1016/J.CLNU.2009.07.008 [retrieved on 2009-08-08]
- Wittmann Anke ET AL: "Sphingomyelin Synthase 1 Is Essential for Male Fertility in Mice", Plos one, 27 October 2016 (2016-10-27), XP093008329, DOI: https://doi.org/10.1371/journal.pone.01642 98 Retrieved from the Internet: URL:https://journals.plos.org/plosone/arti cle/file?id=10.1371/journal.pone.0164298&t ype=printable [retrieved on 2022-12-14]

## Beschreibung

Die Erfindung betrifft Omega-3-Fettsäure(n) zur Verwendung bei der therapeutischen Behandlung des menschlichen oder tierischen Körpers.

Sphingolipide, insbesondere Sphingomyeline, haben in Zellmembranen von Säugetieren verschiedene Funktionen, die u.a. Aufbau, Adhäsion und Signalübermittlung der Zellen und insbesondere auch den Zellkern betreffen. Durch ein Ungleichgewicht der Lipidzusammensetzung verursachte Erkrankungen können folglich durch eine Beeinträchtigung des Sphingolipid-Metabolismus im tierischen oder menschlichen Körper hervorgerufen werden.

In der Veröffentlichung "Omega-3 Fats Provide Powerful Fertility Benefits" von Mike Barrett unter natura Isociety .com/omega-3-fats-provide-powerful- fertiliy-benefits/ ist bekannt, dass die Einnahme von Omega-3-Fettsäuren Vorteile für die männliche Fruchtbarkeit bringen kann, da sie eine wichtige Rolle bei der Akrosombildung spielen.

Die Veröffentlichung "Relationship of omega-3 and omega-6 fatty acids with semen characteristics, and anti-oxidant status of seminal plasma: A comparison between fertile and infertile men" von Sarafinejad et al., Clinical Nutrition 29 (2010) 100-105, beschreibt eine Studie über die Wirkung der Verwendung von Omega-3-Fettsäure und von Omega-6-Fettsäure auf Sameneigenschaften und Samenplasma.

Die Veröffentlichung "Docosahexanoic acid antagonizes TNF-alpha-induced necroptosis by attenuating oxidative stress, ceramide production, lysosomal dysfunction, and autophagic feat" von PACHECO FABIO ET AL., INFLAMMATION RESEARCH, 63, (2014-08-06), 859-871 offenbart, dass DHA (beanspruchte Verbindung) den TNF-alpha-induzierten Sphingolipid-Stoffwechsel in vitro hemmt.

Der vorliegenden Erfindung liegt die Aufgabe zugrunde, ein Mittel zur Verfügung zu stellen, mit dem sich durch beeinträchtigten Sphingolipid-Metabolismus bedingte Erkrankungen behandeln lassen.

Erfindungsgemäß wird diese Aufgabe gelöst durch Verwendung bei einer Erkrankung, die durch einen beeinträchtigten Sphingolipid-Metabolismus und
a) eine beeinträchtigte Sphingolipid-Synthese oder/und einen übermäßigen Sphingolipid-Abbau,
b) eine fehlende, reduzierte oder/und fehlerhafte Bildung eines Sphingomyelin Synthase Enzyms im Körper oder/und
c) einen mangelnden Sphingolipid-Anteil im Blut oder/und in einem Hoden des Körpers
verursacht ist, wobei die Omega-3-Fettsäure(n) mehrfach ungesättigte Omega-3-Fettsäure(n) umfasst bzw. umfassen.

Überraschend hat sich gezeigt, dass die Beeinträchtigung des Sphingolipid-Metabolismus, d.h. insbesondere die Sphingolipid-Synthese und der Abbau von Sphingolipiden, zumindest in gewissem Umfang durch die Verwendung der Omega-3- und/ oder Omega-6-Fettsäure(n) behoben oder gelindert werden kann.

Eine Erklärung dafür ist, dass die Zusammensetzung der Sphingolipide, insbesondere der Sphingomyeline, beispielsweise im Hoden einen außergewöhnlich großen Anteil an langkettigen, mehrfach ungesättigten Fettsäuren wie Omega-3- oder Omega-6-Fettsäuren, z.B. Docosahexaensäure (DHA), aufweist und dass deshalb zur Sphingolipid-Synthese Quellen für solche Fettsäuren vorhanden sein müssen. Damit der zur Sphingolipid-Synthese benötigte Anteil bereitgestellt werden kann, müssen solche Fettsäuren zum einen durch die Nahrung aufgenommen werden, zum anderen werden kurzkettige Fettsäuren in langkettige, mehrfach ungesättigte Fettsäuren umgewandelt. Werden diese Fettsäuren dagegen nicht in ausreichender Menge zur Verfügung gestellt, ist die Sphingolipid-Synthese beeinträchtigt.

Stellt der Körper trotz an sich ausreichend ergiebiger Quellen keine ausreichende Menge an Sphingolipiden zur Verfügung, lässt sich durch die Verwendung die Produktivität der Sphingolipid-Synthese steigern. Ein Grund dafür ist, dass ein größerer Produktionsdruck vorliegt, wenn größere Mengen an den Omega-3- bzw. Omega-6-Fettsäuren zur Verfügung stehen.

Hat der beeinträchtigte Sphingolipid-Metabolismus zur Folge, dass die Sphingolipide im Körper zu schnell abgebaut werden und aus diesem Grund nicht in ausreichender Menge zur Verfügung stehen, lässt sich dies durch die Verwendung und die dadurch gesteigerte Sphingolipid-Bildung ausgleichen.

Durch die Verwendung der Omega-3- und/oder Omega-6-Fettsäure(n), vorzugsweise durch intravenöse oder orale Verabreichung, werden ausreichend große Mengen der langkettigen, mehrfach ungesättigten Fettsäuren zur Nutzung bereitgestellt und die Erkrankungen, die durch den beeinträchtigten Sphingolipid-Metabolismus verursacht wird, können geheilt oder die durch die Erkrankung verursachten Leiden gelindert werden. Eine solche Erkrankung kann männliche Unfruchtbarkeit, Speicherkrankheiten, Demenzerkrankungen, insbesondere die Alzheimerkrankheit, die Niemann-Pick-Erkrankung, oder Multiple Sklerose umfassen. Ferner kommen als Erkrankung Krebserkrankungen, die Tay-Sachs-Krankheit, die Sandhoff- Krankheit, die Tay-Sachs AB Variante, die Gaucher-Krankheit, die Fabry-Krankheit, die Krabbe- Krankheit, G_{M1} Gangliosidose und metachromatische Leukodystrophie in Betracht.

Die Sphingolipide und die sich davon ableitenden Ganglioside haben eine wichtige Funktion insbesondere bei der Spermatogenese. In Zellmembranen von Spermien bilden Phospholipide mit mehrfach ungesättigten Fettsäuren, insbesondere sehr langkettigem Sphingomyelin, einen großen Anteil an Lipiden. Die Erfinder haben herausgefunden, dass ein Mangel an den langkettigen, mehrfach ungesättigten Fettsäuren zu Veränderungen von Spermatozyten und Spermatiden, insbesondere zu Fehlreifungen, führen kann und dass die Veränderungen männliche Unfruchtbarkeit verursachen. Es hat sich überraschend gezeigt, dass sich die Ausbildung der Veränderungen durch die erfindungsgemäße Verwendung nicht nur vermeiden lässt, sondern dass sich bereits gebildete Veränderungen wieder zurückbilden.

Die erfindungsgemäße Verwendung eignet sich insbesondere zur Behandlung bei fehlender, reduzierter oder/und fehlerhafter Bildung eines Sphingomyelin Synthase Enzyms, insbesondere von Sphingomyelin Synthase 1 (SMS1) oder/und Sphingomyelin Synthase 2 (SMS2), im Körper. SMSI und SMS2 katalysieren eine de novo-Synthese von Sphingomyelin und Diacylglycerol (DAG) aus Ceramiden und Phoshpatidylcholinen, während das homologe Enzym SMSr (sphingomyelin synthase-related) keine Sphingomyelin-Synthase-Akitivität aufweist. SMS1 ist im Cis-Golgi-Apparat angeordnet und für die Bildung des Sphingomyelin verantwortlich. Allerdings findet sich aufgrund von vesikelvermittelndem Stoffaustausch zwischen dem Golgi-Apparat und Plasmamembranen die höchste Konzentration an Sphingomyelin und anderen komplexen Sphingolipiden in äußeren Bereichen der Plasmamembranen, in denen die Sphingolipide die eingangs genannten Funktionen erfüllen. An den Plasmamembranen werden diese Funktionen durch SMS2 vermittelt, das eine wichtige Rolle zur Kontrolle der Ceramid-Levels der Plasmamembrane spielt.
Die genannten Verschorfungen treten bei beeinträchtigter Bildung der SMSI auf, weil SMS1 eine wichtige Rolle bei der Aufrechterhaltung der intrazellulären Sphingomyelinhomeostase spielt, welche einen Sphingomyelinvorrat bildet, aus dem auch die Plasmamembrane versorgt werden. Ein Ungleichgewicht im Lipidmetabolismus, das durch die genannte Beeinträchtigung hervorgerufen wird, betrifft deshalb die intrazelluläre Lipidzusammensetzung und die Lipidzusammensetzung an der Zelloberfläche, welche Proteintransport, enzymatische Aktivität und lipidbasierte Zellsignalübertragung ändern können. Durch die erfindungsgemäße Verwendung lässt sich der Lipidmetabolismus überraschenderweise wieder ins Gleichgewicht oder zumindest näher an das Gleichgewicht bringen.

Als besonders wirksam hat sich die Verwendung bei einem Defekt des Sphingomyelin Synthase 1 Gens und/oder des Sphingomyelin Synthase 2 Gens erwiesen. Die Erfinder haben festgestellt, dass ein Defekt des jeweiligen Gens zu einer altersabhängigen Reduzierung verschiedener langkettiger, mehrfach ungesättigter Phosphatidylcholine, Lysophosphatidylcholine und Sphingolipide im Hoden führt, die mit fortschreitendem Alter auftritt und eine altersbedingte Unfruchtbarkeit zur Folge haben kann. Durch die Verwendung der Omega-3- und/oder Omega-6-Fettsäure(n) kann die Auswirkungen eines Defekts am Sphingomyelin Synthase 1 Gen und/oder des Sphingomyelin Synthase 2 Gen überraschenderweise ausgeglichen werden, sodass die genannte Reduzierung nicht mehr besteht. Die genannten Veränderungen bilden sich durch die Verwendung zurück und die Unfruchtbarkeit kann geheilt werden.

In einer Ausgestaltung der Erfindung umfassen die Omega-3- und/oder Omega-6-Fettsäure(n) hoch ungesättigte Omega-3- und/oder Omega-6-Fettsäure(n), vorzugsweise EPA, DHA, DPA, ETA, 21:5n3 und/oder SDA.

Zweckmäßigerweise werden die Omega-3- und/oder Omega-6-Fettsäure(n) als freie Fettsäuren oder in gebundener Form, insbesondere als Ester oder Salze, verwendet. Vorzugsweise ist das Mittel frei von oder arm an anderen Fettsäuren als Omega-3und/oder Omega-6-Fettsäure(n).

In einer Ausführungsform der Erfindung werden die Omega-3- und/oder Omega-6-Fettsäure(n), insbesondere beim Menschen, in einer Dosierung von mehr als 0,5 g/Tag, vorzugsweise 1 bis 3 g/Tag, verabreicht. Als besonders wirksam hat sich eine Verabreichung von 2 bis 3 g/Tag erwiesen. Eine Verabreichung von mehr als 5 g/Tag dagegen hat sich als nicht zweckmäßig erwiesen.

Die Omega-3- und/oder Omega-6-Fettsäure(n) werden bevorzugt intravenös oder oral verabreicht. Vorzugsweise werden sie in Form von Kapseln, Tabletten oder/und Flüssigkeiten verabreicht. Eine bestimmte Dosierungsmenge des Mittels wird pro Tag zweckmäßigerweise ein- bis dreimalig, vorzugsweise zweimalig, zugeführt. In einer besonders bevorzugten Ausführungsform der Erfindung ist/sind die Omega-3- und/ oder Omega-6-Fettsäure(n) aus Fisch, insbesondere Fischöl, und/oder Mikroorganismen, insbesondere Algen, gewonnen worden.

Nachfolgend wird die Wirkung der Verwendung anhand von Beispielen und der beigefügten Tabellen, Grafiken und Abbildungen, die sich auf die Beispiele beziehen, näher erläutert. Es zeigen:
- Fig. 1: eine Tabelle mit Ergebnissen einer massenspektrometrischen Analyse von Phosphatidylcholinfettsäurenzusammensetzung in Hoden von Mäusen,
- Fig. 2: Hodenphänotypen von Mäusen,
- Fig. 3: eine Grafik betreffend die Wurfgrößen von Mäusen nach Verpaarung,
- Fig. 4: eine Grafik betreffend die Dauer bis zum ersten Wurf von Mäusen nach Verpaarung, und
- Fig. 5: Hämatoxylin und Eosin Färbung der Hoden.

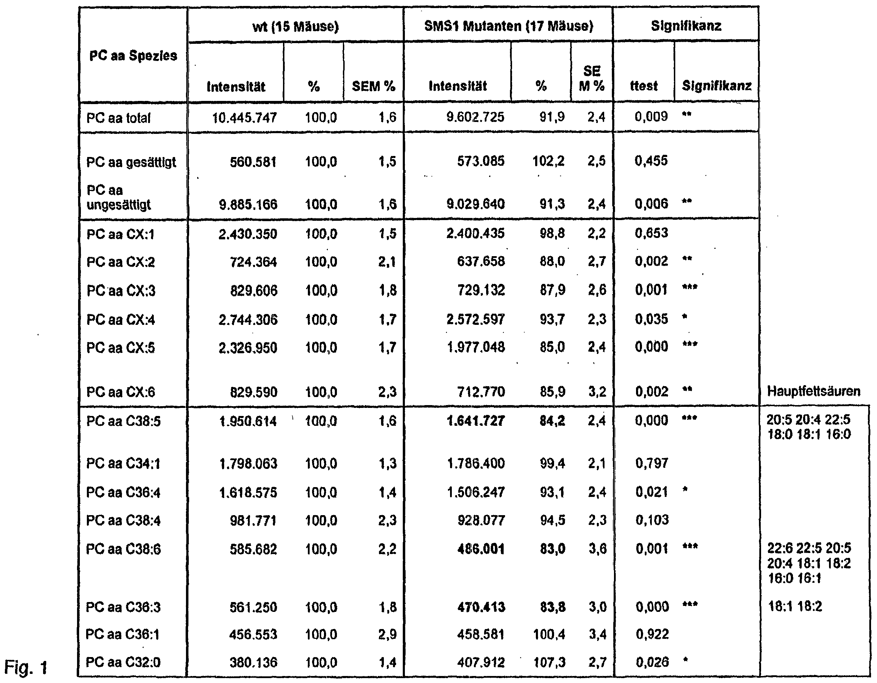

Bei Mäusen des Stammes C57BI6J wurde die Funktion der Sphingomyelin Synthase 1 (SMS1) mittels der Gen-Trap Methode entsprechend eines knock-outs vollständig oder zumindest weitgehend entfernt. Diese Mäuse werden nachfolgend als genmodifizierte Mäuse bezeichnet. Dies führte im Vergleich zu einer Referenzgruppe von nicht genmanipulierten Mäusen des Stammes C57BI6J, die nachfolgend als "wild-type-Mäuse" bezeichnet werden, zu einer Verringerung von Sphingomyelin um 21,1% (p<0.0001) im Hoden und insbesondere zu einer Verringerung des Anteils an mehrfach ungesättigten Fettsäuren, insbesondere von mehrfach ungesättigten Omega-3 und Omega-6 Fettsäuren. Die Verringerung der Lipide und der Fettsäuren beschränkt sich nicht auf die Sphingomyeline, sondern betrifft auch andere Phospholide. Wie der Tabelle in Fig. 1 u.a. zu entnehmen ist, betrug die Verringerung von Phosphatidylcholin C38:6, welches mehrfach ungesättigte Fettsäuren wie DHA, EPA und Arachidonsäure enthält, 17% (p<0.001).

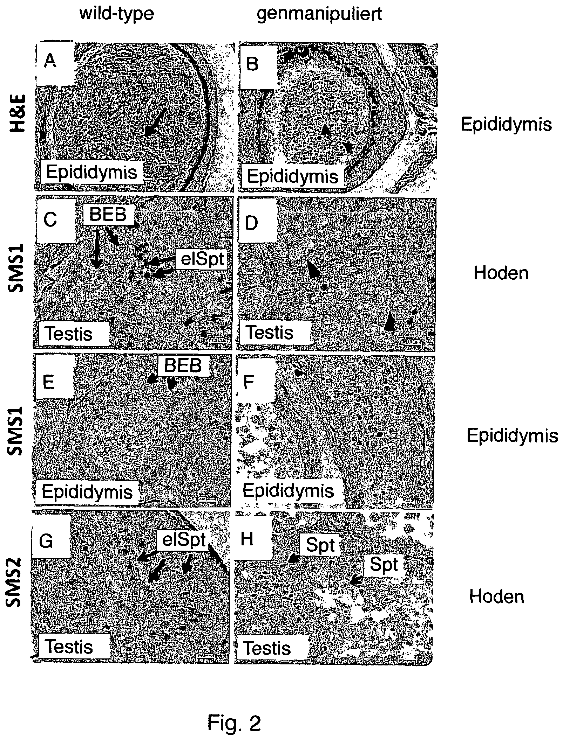

Fig. 2 zeigt pathologische Veränderungen der Hoden der genmodifzierten Mäuse, die sich einhergehend mit der Verringerung der Sphingomyeline eingestellt haben. In Fig. 2A und 2B zeigen eine Hämatoxylin- und Eosinfärbung ("H&E-Färbung") von Hoden einer der wild-type-Mäuse (Fig. 2A) und einer der genmanipulierten Mäuse (Fig. 2B). Die Epididymiden der Hoden zeigen eine Ansammlung von veränderten Zellen bei der genmodifizierten Maus (schwarze Pfleilköpfe in Fig. 2B) im Verhältnis zu den Reifen Spermatozoen der wild-type-Maus (schwarze Pfeile in Fig. 2A).

Eine Immunhistochemie zeigt bei den genmodifizierten Mäusen (Fig. 2D) nahe der Interzellulärraumes einschließlich der Blut-Hodenschranke (BTB) und in Akrosomen sich entwickelnder Spermatozyten. Pfeilköpfe in Fig. 2D zeigen auf große Vakuolen innerhalb von Tuben bei den genmodifizierten Maus, welche einen besonders stark ausgeprägten Phänotyp wiedergeben.

Fig. 2E und 2F zeigen in einer Immunhistochemie eine SMS1 Expression in Zellen, die den Hauptteil des Epithelium darstellen. Der Vergleich zwischen der wild-type-Maus (Fig. 2E) und der genmodifizierten Maus (Fig. 2F) zeigt, dass bei der genmodifizierten Maus die SMS1 Expression nahezu vollständig fehlt.

Wie Fig. 2G und 2H zu entnehmen ist, ist die Immunhistochemie für SMS2 bei der wild-type-Maus normal verteilt, aber bei der genmodifizierten Maus punktförmig reduziert auf Spermatidenzellen.

Als Konsequenz des veränderten Sphingolipidmetabolism und der daraus hervorgegangenen fehlerhaften Lipidzusammensetzung und Hodenpathologie trat bei männlichen Mäusen Unfruchtbarkeit auf.

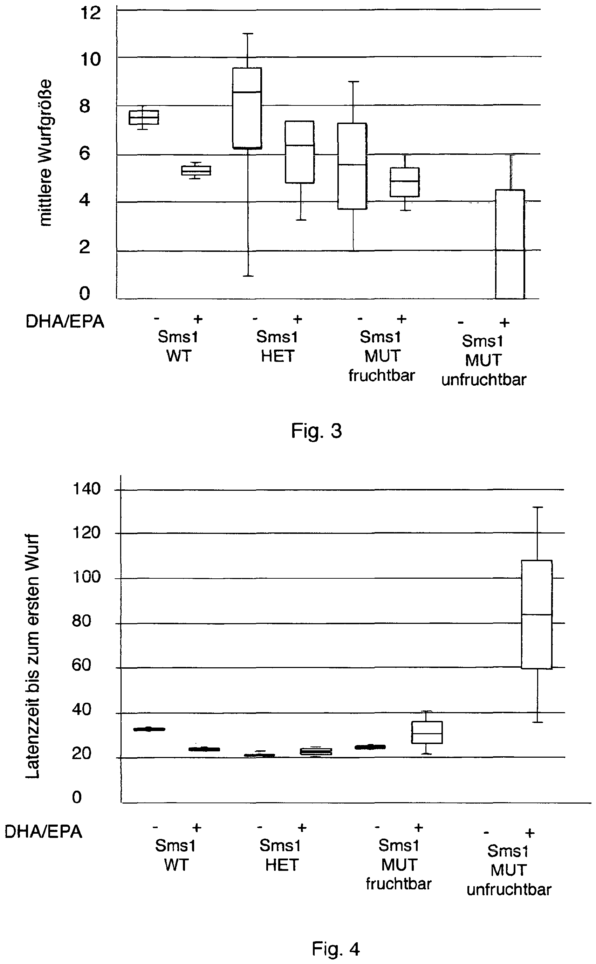

Zunächst ist die Fruchtbarkeit von männlichen wild-type-, heterozygoten und genmanipulierte Mäusen untersucht worden, indem männliche Mäuse mit einer 11 Wochen alten weiblichen Maus des Stammes C57BI/6J verpaart wurden. Die Ergebnisse dieser Untersuchung finden sich in der Grafik in Fig. 3 anhand von erreichten Wurfgrößen unter Sms1WT (Verpaarung der wild-type-Mäuse), Sms1HET (Verpaarung der heterozygoten Mäuse) und Sms1MUT (Verpaarung der genmanipulierten Mäuse), jeweils gekennzeichnet durch "-". Nach dieser Untersuchung wurde regelmäßig eine Mischung aus DHA/EPA manuell verfüttert (Mischung aus Ethylester Mischung mit DHA (38%) und EPA (46), 9mg je Maus täglich). Männliche wild-type Mäuse, heterozygote und genmanipulierte Mäuse, deren Fruchtbarkeit zuvor untersucht worden war, wurden für 13 Wochen erneut verpaart und wurden auch währenddessen weiter mit DHA/EPA gefüttert. Die Ergebnisse sind der zweiten Verpaarung in der Grafik in Fig. 3 jeweils mit "+" gekennzeichnet.

Wie Fig. 3 zeigt, sind die genmanipulierten und zunächst unfruchtbaren Mäuse durch die Einnahme der Mischung fruchtbar geworden. Aus Fig. 4, die die Dauer nach Verpaarung bis zum Wurf zeigt, geht hervor, dass wild-typ Mäuse (WT), heterozygote (HET) und fertile genmanipulierte Mäuse (MUT) auch bei Verfütterung des Mittels ähnlich lange Zeit bis zum ersten Wurf benötigt haben. Die infertilen und genmanipulierten, mit der genannten Mischung gefütterten und dadurch von der Infertilität geheilten Mäuse dagegen benötigten deutlich mehr Zeit.

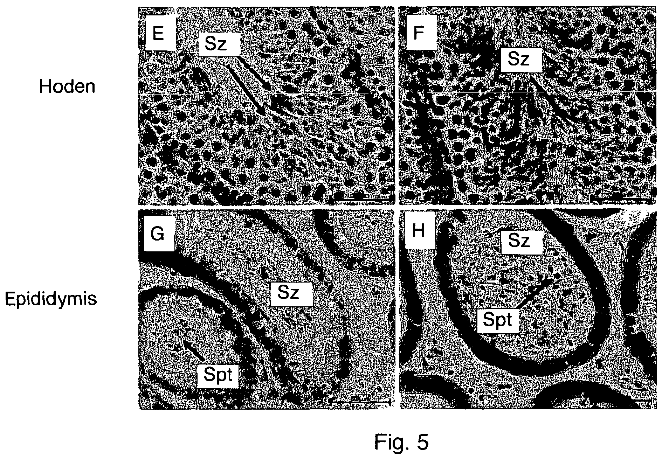

Ferner führte, wie aus Fig. 5 hervorgeht, die Anreicherung der normalen Nahrung mit DHA und EPA zu einer Verbesserung der Hodenpathologie der genmanipulierten Mäuse. Zuvor infertile genmanipulierte Mäuse, die nach der DHA/EPA Behandlung Nachkommen gezeugt hatten, zeigen deutlich geringere pathologische Veränderungen in den Hoden (vgl. Fig. 4E und Fig. 4F) als auch in den Epididymiden (vgl. Fig. 4G und Fig. 4H) im Vergleich zu den wild-type-Mäusen. Pfeile in Fig. 4 zeigen auf elongierte Spermatiden (Spt) und Spermatozoen (Sz). Wie erkennbar ist, dominieren die Spermatozoen gegenüber den Spermatiden. Dies stimmt mit dem Reifezyklus für Spermien und einer Reduktion der Hodenpathologie überein.

## Patentansprüche

1. Omega-3-Fettsäure(n) zur Verwendung bei der therapeutischen Behandlung des menschlichen oder tierischen Körpers,
**gekennzeichnet durch** Verwendung bei einer Erkrankung, die durch einen beeinträchtigten Sphingolipid-Metabolismus und
a) eine beeinträchtigte Sphingolipid-Synthese oder/und einen übermäßigen Sphingolipid-Abbau,
b) eine fehlende, reduzierte oder/und fehlerhafte Bildung eines Sphingomyelin Synthase Enzyms im Körper oder/und
c) einen mangelnden Sphingolipid-Anteil im Blut oder/und in einem Hoden des Körpers
verursacht ist,
wobei die Omega-3-Fettsäure(n) mehrfach ungesättigte Omega-3-Fettsäure(n) umfasst bzw. umfassen.

2. Omega-3-Fettsäure(n) zur Verwendung nach Anspruch 1,
**dadurch gekennzeichnet,**
**dass** das Enzym Sphingomyelin Synthase 1 oder Sphingomyelin Synthase 2 ist.

3. Omega-3-Fettsäure(n) zur Verwendung nach Anspruch 1 oder 2,
**gekennzeichnet durch** eine Verwendung bei einem Defekt des Sphingomyelin Synthase 1 Gens und/oder des Sphingomyelin Synthase 2 Gens.

4. Omega-3-Fettsäure(n) zur Verwendung nach einem der Ansprüche 1 bis 3,
**dadurch gekennzeichnet,**
**dass** die Omega-3-Fettsäure(n) EPA, DHA, DPA, ETA, 21:5n3 und/oder SDA, umfassen.

5. Omega-3-Fettsäure(n) zur Verwendung nach einem der Ansprüche 1 bis 4,
**dadurch gekennzeichnet,**
**dass** die Omega-3-Fettsäure(n) als freie Fettsäuren oder in gebundener Form, insbesondere als Ester oder Salze, verwendet werden.

6. Omega-3-Fettsäure(n) zur Verwendung nach einem der Ansprüche 1 bis 5,
**dadurch gekennzeichnet,**
**dass** die Omega-3-Fettsäure(n) in einer Dosierung von mehr als 0,5 g/Tag, vorzugsweise 1 bis 3 g/Tag, verabreicht werden.

## Claims

1. Omega-3 fatty acid(s) for use in the therapeutic treatment of the human or animal body,
**characterized by** use in a disease caused by impaired sphingolipid metabolism and
a) impaired sphingolipid synthesis or/and excessive sphingolipid degradation,
b) absent, reduced or/and incorrect formation of a sphingomyelin synthase enzyme in the body or/and
c) an insufficient sphingolipid content in the blood or/and in a testis of the body,
wherein the omega-3 fatty acid(s) comprise(s) polyunsaturated omega-3 fatty acid(s).

2. Omega-3 fatty acid(s) for use according to Claim 1,
**characterized in that**
the enzyme is sphingomyelin synthase 1 or sphingomyelin synthase 2.

3. Omega-3 fatty acid(s) for use according to Claim 1 or 2,
**characterized by** use in a defect of the sphingomyelin synthase 1 gene and/or the sphingomyelin synthase 2 gene.

4. Omega-3 fatty acid(s) for use according to any of Claims 1 to 3,
**characterized in that**
the omega-3 fatty acid(s) comprise EPA, DHA, DPA, ETA, 21:5n3 and/or SDA.

5. Omega-3 fatty acid(s) for use according to any of Claims 1 to 4,
**characterized in that**
the omega-3 fatty acid(s) are used as free fatty acids or in bound form, in particular as esters or salts.

6. Omega-3 fatty acid(s) for use according to any of Claims 1 to 5,
**characterized in that**
the omega-3 fatty acid(s) are administered in a dose of more than 0.5 g/day, preferably 1 to 3 g/day.

## Revendications

1. Acide(s) gras oméga-3 destiné(s) à être utilisé(s) dans le traitement thérapeutique du corps humain ou animal, caractérisé(s) par l'utilisation lors d'une maladie qui est provoquée par un métabolisme compromis des sphingolipides et
a) une synthèse compromise des sphingolipides et/ou une dégradation excessive des sphingolipides,
b) une formation manquante, réduite et/ou erronée d'une enzyme de sphingomyéline synthase dans le corps et/ou
c) une proportion de sphingolipides faisant défaut dans le sang et/ou dans un testicule du corps,
le(s) acide(s) gras oméga-3 comprenant un/des acide(s) gras oméga-3 polyinsaturés.

2. Acide(s) gras oméga-3 destiné(s) à être utilisé(s) selon la revendication 3, caractérisé(s) en ce que l'enzyme est la sphingomyéline synthase 1 ou la sphingomyéline synthase 2.

3. Acide(s) gras oméga-3 destiné(s) à être utilisé(s) selon la revendication 1 ou 2, caractérisé(s) par une utilisation lors d'un défaut du gène de la sphingomyéline synthase 1 et/ou du gène de la sphingomyéline synthase 2.

4. Acide(s) gras oméga-3 destiné(s) à être utilisé(s) selon l'une des revendications 1 à 3, caractérisé(s) en ce que le(s) acide(s) gras oméga-3 comprennent l'EPA, le DHA, le DPA, l'ETA, le 21:5n3 et/ou le SDA.

5. Acide(s) gras oméga-3 destiné(s) à être utilisé(s) selon l'une des revendications 1 à 4, caractérisé(s) en ce que le(s)acide(s) gras oméga-3 est/sont utilisé(s) sous forme d'acide(s) gras libre(s) ou sous forme liée, en particulier sous forme d'ester(s) ou de sel(s).

6. Acide(s) gras oméga-3 destiné(s) à être utilisé(s) selon l'une des revendications 1 à 5, caractérisé(s) en ce que le(s) acide(s) gras oméga-3 est/sont administré(s) en une posologie de plus de 0,5 g/jour, de préférence de 1 à 3 g/jour.
